# EUROPEAN PATENT SPECIFICATION

(11) **EP 3 130 328 B1**
(45) Date of publication and mention of the grant of the patent: **25.11.2020**
(21) Application number: 16183836.2
(22) Date of filing: 11.08.2016
(51) Int. Cl.: A61K 31/137, A61K 8/44, A61K 31/195, A61Q 11/00

(54) **ORAL CARE COMPOSITION FOR RELIEVING GUM BLEEDING**
MUNDPFLEGEMITTEL ZUR LINDERUNG VON ZAHNFLEISCHBLUTEN
COMPOSITION DE SOINS BUCCO-DENTAIRES POUR SOULAGEMENT DU SAIGNEMENT DES GENCIVES

(30) Priority: 13.08.2015 CN 201510587338
(43) Date of publication of application: 15.02.2017
(73) Proprietor: Colgate-Palmolive Company, New York, NY 10022 (US)
(72) Inventor: XU, Shao Peng, Guangzhou, Guangdong (CN); YAN, Peng, Guangzhou, Guangdong (CN)
(74) Representative: Wibbelmann, Jobst

(56) References cited:
- WO-A1-00/37071
- DD-A1- 250 465

## Description

### BACKGROUND

Bleeding gums are a common oral problem, and can be a sign of gingivitis and other oral conditions and may lead to serious medical problems if not addressed. Bleeding of the gums is caused by a build-up of plaque, which generates toxins that cause inflammation of the soft gum tissue. Many dentifrice products have been launched worldwide claiming gum bleeding relief efficacy, such as Yun Nan Bai Yao Toothpaste in China and Paradontox Toothpaste in Phillippines. Antibacterial agents have been used in in oral care products to reduce plaque and gingivitis, and hence to relieve gum bleeding. Although the coagulant tranexamic acid has been used in oral care products to treat bleeding gums, its use in such compositions is limited due to potential thromboembolic risk and other drug interactions. It therefore would be desirable to provide an alternative oral care composition which can treat or inhibit bleeding of the gums.

### BRIEF SUMMARY

The present invention provides an oral care composition for use in a method of reducing or inhibiting gum bleeding comprising 4-(aminomethyl)benzoic acid in an amount of from 0.1 weight % to 0.5 weight %, based on the total weight of the composition.

Preferably, the 4-(aminomethyl)benzoic acid is present in an amount of 0.3 weight %, based on the total weight of the composition.

Optionally, the composition is free from tranexamic acid.

Optionally, the oral care composition further comprises a fluoride ion source, an abrasive, an antibacterial agent, an anticalculus agent, or an antisensitivity agent, or a combination of any two or more thereof.

Optionally, the oral care composition further comprises a diluent, a foam modulator, a thickening agent, a viscosity modifier, a humectant, a sweetener, a flavorant or a pigment, or a combination of any two or more thereof.

Optionally, the composition is a toothpaste, tooth gel, mouth wash, mouth rinse, spray, strip, chewing gum, lozenge, chewable tablet, dissolvable tablet, or confectionary item.

The method of reducing or inhibiting gum bleeding comprises applying an effective amount of said oral care composition to the oral cavity of a subject in need thereof.

Further areas of applicability of the present invention will become apparent from the detailed description provided hereinafter.

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material. Unless otherwise specified, all ratios expressed herein and elsewhere in the specification should be understood to refer to ratios by weight.

As discussed above, it would be desirable to provide oral care compositions which can treat or inhibit bleeding of the gums.

The present invention therefore provides an oral care composition for use in a method of reducing or inhibiting gum bleeding comprising 4-(aminomethyl)benzoic acid in an amount of from 0.1 weight % to 0.5 weight %, based on the total weight of the composition. The present inventors have surprisingly found that 4-(aminomethyl)benzoic acid has a similar clotting time to tranexamic acid in the In-vitro Prothrombin Time (PT) Test (see Examples, below), but has been found to be safe for oral care applications.

In some embodiments, the oral care composition is free from tranexamic acid. By "free from tranexamic acid", it is meant that the composition contains less than 0.05 weight % tranexamic acid, contains less than 0.01 weight % tranexamic acid, contains substantially no tranexamic acid, or contains no tranexamic acid (i.e. contains 0 weight % tranexamic acid).

In certain embodiments, the 4-(aminomethyl)benzoic acid is present in an amount of from 0.2 weight % to 0.4 weight %, or 0.3 weight %, based on the total weight of the composition.

In some embodiments, the oral care composition is a toothpaste, tooth gel, mouth wash, mouth rinse, spray, strip, chewing gum, lozenge, chewable tablet, dissolvable tablet, or confectionary item.

In various embodiments, the oral care composition further comprises a fluoride ion source, an abrasive, an antibacterial agent, an anticalculus agent, or an antisensitivity agent, or a combination of any two or more thereof. Examples of fluoride ion sources which may be used include sodium fluoride and sodium monofluorophosphate. Examples of abrasives which may be used include silica abrasives. Examples of antibacterial agents which could be used include triclosan, zinc oxide and zinc citrate. Examples of anticalculus agents which could be used include tetrapotassium pyrophosphate and tetrasodium pyrophosphate. An example of an antisensitivity agent which could be used is potassium nitrate.

The oral care composition may also further comprise a diluent, a foam modulator, a thickening agent, a viscosity modifier, a humectant, a surfactant, a sweetener, a flavorant or a pigment, or a combination of any two or more thereof. Examples of diluents which could be used include water and ethanol. Examples of foam modulators which could be used include polyethylene glycols. Examples of thickening agents which could be used include cellulose gums. Examples of viscosity modifiers which could be used include clays and silicas. Examples of humectants which could be used include polyhydric alcohols. Examples of surfactants which could be used include anionic surfactants, nonionic surfactants and cationic surfactants.

### EXAMPLES

### Example 1

An In-vitro Prothrombin Time (PT) Test has been designed to test the coagulant efficacy (see Luz, L. et al., International Journal of Biological Macromolecules, 58 (2013), p. 31-36).

The coagulant efficacy of the compositions and ingredients of the present invention was tested against that of tranexamic acid and two controls: deionized (DI) water, and a solution of 0.9 weight % sodium chloride in water (hereinafter referred to as "saline solution"). The experimental methodology used was:

### 1) Sample preparation

When testing the "Coagulant Ingredients" tranexamic acid and 4-(aminomethyl)benzoic acid in Table 1, below, the active ingredient was mixed with water at a 1:100 ratio by weight, to give a concentration of 1.0 weight % (to one decimal place) of the ingredient under test. When testing the "Toothpastes" in Table 2, below, a toothpaste slurry was prepared by mixing 1 part of the toothpaste under test with 2 parts DI water (all parts being parts by weight); then the supernatant was obtained by centrifuging the slurry for 20 mins at 8000 rpm.

### 2) Prothrombin Time (PT) Test

0.1mL of experimental animal plasma was placed in each testing tube (animal blood samples used in the experiment were waste products, and the animals were otherwise used for food). 0.05mL of the test samples (0.05mL of DI water or saline solution in control group) were added to separate tubes and mixed with the plasma. The mixtures were then heated in a water bath at 37°C for 3min, then 0.2mL of thromboplastin (which had been preheated to 37°C) was added to each testing tube and the contents mixed. A stopwatch was started immediately upon adding the thromboplastin to record the coagulation time (Prothrombin Time). The experiment was repeated 10 times for each sample, and the mean Prothrombin Time calculated. The results are shown in Tables 1 and 2, below:

**Table 1**

| Sample | Coagulant Ingredient | | | |
|---|---|---|---|---|
| | DI water | Saline solution | 4-(aminomethyl) benzoic acid (1 weight %*) | Tranexamic acid (1 weight %) |
| Prothrombin Time (s) | 19.37 | 17.40 | 11.14 | 11.66 |

| | | | | |
|---|---|---|---|---|
| *not claimed | | | | |

**Table 2**

| Sample | Toothpaste | |
|---|---|---|
| | Control toothpaste | Toothpaste w. 0.3 weight % 4-(aminomethyl) benzoic acid |
| Prothrombin Time (s) | 22.69 | 10.57 |

As can be seen in Table 1, 4-(aminomethyl) benzoic acid has significantly lower clotting time than the controls (DI water and saline solution). No significant difference in clotting time was observed between tranexamic acid and 4-(aminomethyl) benzoic acid.

In the toothpaste sample study in Table 2, it was shown that the toothpaste containing 0.3 weight % 4-(aminomethyl)benzoic acid has a significantly lower Prothrombin time than the control toothpaste.

These results indicate that 4-(aminomethyl) benzoic acid can accelerate the in-vitro coagulation process, and can be applied in toothpaste to deliver gum bleeding relief efficacy.

### Example 2

An example of a toothpaste containing 4-(aminomethyl)benzoic acid is given in Table 3, below:

**Table 3**

| **Ingredient** | **Weight %** |
|---|---|
| 4-(aminomethyl)benzoic acid | 0.1 - 0.5 |
| Humectants | 30 - 50 |
| Thickening agents | 5 - 15 |
| Abrasives | 5 - 20 |
| Surfactants | 5 - 15 |
| Fluoride ion source | 0.1 - 1 |
| Anticalculus agents | 0.1 - 5 |
| Antibacterial agents and preservatives | 0.5 - 5 |
| Water and minors | 10 - 40 |

## Claims

1. An oral care composition for use in a method of reducing or inhibiting gum bleeding comprising 4-(aminomethyl)benzoic acid in an amount of from 0.1 weight % to 0.5 weight %, based on the total weight of the composition.

2. The oral care composition for use according to claim 1, wherein the 4-(aminomethyl)benzoic acid is present in an amount of from 0.2 weight % to 0.4 weight %, based on the total weight of the composition.

3. The oral care composition for use according to claim 2, wherein the 4-(aminomethyl)benzoic acid is present in an amount of about 0.3 weight %, based on the total weight of the composition.

4. The oral care composition for use according to any preceding claim, wherein the composition is free from tranexamic acid.

5. The oral care composition for use according to any preceding claim, further comprising a fluoride ion source, an abrasive, an antibacterial agent, an anticalculus agent, or an antisensitivity agent, or a combination of any two or more thereof.

6. The oral care composition for use according to any preceding claim, further comprising a diluent, a foam modulator, a thickening agent, a viscosity modifier, a humectant, a sweetener, a flavorant or a pigment, or a combination of any two or more thereof.

7. The oral care composition for use according to any preceding claim, wherein the composition is a toothpaste, tooth gel, mouth wash, mouth rinse, spray, strip, chewing gum, lozenge, chewable tablet, dissolvable tablet, or confectionary item.

8. The oral care composition for use according to any preceding claim, wherein the method comprises applying an effective amount of said oral care composition to the oral cavity of a subject in need thereof.

## Patentansprüche

1. Mundpflegezusammensetzung zur Verwendung in einem Verfahren zum Verringern oder Verhindern von Zahnfleischbluten, umfassend 4-(Aminomethyl)benzoesäure in einer Menge von 0,1 Gewichts-% bis 0,5 Gewichts-%, bezogen auf das Gesamtgewicht der Zusammensetzung.

2. Mundpflegezusammensetzung zur Verwendung gemäß Anspruch 1, wobei die 4-(Aminomethyl)benzoesäure in einer Menge von 0,2 Gewichts-% bis 0,4 Gewichts-%, bezogen auf das Gesamtgewicht der Zusammensetzung, vorhanden ist.

3. Mundpflegezusammensetzung zur Verwendung gemäß Anspruch 2, wobei die 4-(Aminomethyl)benzoesäure in einer Menge von etwa 0,3 Gewichts-%, bezogen auf das Gesamtgewicht der Zusammensetzung, vorhanden ist.

4. Mundpflegezusammensetzung zur Verwendung gemäß einem beliebigen vorhergehenden Anspruch, wobei die Zusammensetzung frei von Tranexamsäure ist.

5. Mundpflegezusammensetzung zur Verwendung gemäß einem beliebigen vorhergehenden Anspruch, weiterhin umfassend eine Fluoridionenquelle, ein Abrasivmittel, ein antibakterielles Mittel, ein Mittel gegen Zahnstein oder ein Antisensitivitätsmittel oder eine Kombination von zwei beliebigen oder mehreren davon.

6. Mundpflegezusammensetzung zur Verwendung gemäß einem beliebigen vorhergehenden Anspruch, weiterhin umfassend ein Verdünnungsmittel, einen Schaummodulator, ein Verdickungsmittel, einen Viskositätsmodifikator, ein Feuchthaltemittel, ein Süßungsmittel, einen Aromastoff oder ein Pigment oder eine Kombination von zwei beliebigen oder mehreren davon.

7. Mundpflegezusammensetzung zur Verwendung gemäß einem beliebigen vorhergehenden Anspruch, wobei die Zusammensetzung eine Zahnpasta, Zahngel, Mundwasser, Mundspülung, Spray, Streifen, Kaugummi, Pastille, kaubare Tablette, auflösbare Tablette oder Süßwarenartikel ist.

8. Mundpflegezusammensetzung zur Verwendung gemäß einem beliebigen vorhergehenden Anspruch, wobei das Verfahren das Anwenden einer wirksamen Menge der Mundpflegezusammensetzung in der Mundhöhle eines Subjekts, das diese benötigt, umfasst.

## Revendications

1. Composition de soin bucco-dentaire pour une utilisation dans un procédé de réduction ou d'inhibition du saignement des gencives comprenant de l'acide 4-(aminométhyl)benzoïque en une quantité de 0,1 % en poids à 0,5 % en poids, par rapport au poids total de la composition.

2. Composition de soin bucco-dentaire pour une utilisation selon la revendication 1, dans laquelle l'acide 4-(aminométhyl)benzoïque est présent en une quantité de 0,2 % en poids à 0,4 % en poids, par rapport au poids total de la composition.

3. Composition de soin bucco-dentaire pour une utilisation selon la revendication 2, dans laquelle l'acide 4-(aminométhyl)benzoïque est présent en une quantité d'environ 0,3 % en poids, par rapport au poids total de la composition.

4. Composition de soin bucco-dentaire pour une utilisation selon l'une quelconque des revendications précédentes, dans laquelle la composition est exempte d'acide tranexamique.

5. Composition de soin bucco-dentaire pour une utilisation selon l'une quelconque des revendications précédentes, comprenant en outre une source d'ions fluorure, un abrasif, un agent antibactérien, un agent antitartre ou un agent anti-sensibilité, ou une combinaison de deux ou plus de ceux-ci.

6. Composition de soin bucco-dentaire pour une utilisation selon l'une quelconque des revendications précédentes, comprenant en outre un diluant, un modulateur de mousse, un agent épaississant, un modificateur de viscosité, un humectant, un édulcorant, un arôme ou un pigment, ou une combinaison de deux ou plus de celui-ci.

7. Composition de soin bucco-dentaire pour une utilisation selon l'une quelconque des revendications précédentes, dans laquelle la composition est un dentifrice, un gel dentaire, un bain de bouche, un rince-bouche, un aérosol, une bande, un chewing-gum, une pastille, un comprimé à croquer, un comprimé soluble ou un article de confiserie.

8. Composition de soin bucco-dentaire pour une utilisation selon l'une quelconque des revendications précédentes, dans laquelle le procédé comprend l'application d'une quantité efficace de ladite composition de soin bucco-dentaire à la cavité buccale d'un sujet en ayant besoin.
